**Europäisches Patentamt**

**European Patent Office**

**Office européen des brevets**

(19)

(11) Numéro de publication: **0 021 984**
**B1**

(12)     **FASCICULE DE BREVET EUROPEEN**

(45) Date de publication du fascicule du brevet: **25.05.83**

(21) Numéro de dépôt: **80400887.8**

(22) Date de dépôt: **18.06.80**

(51) Int. Cl.³: **A 61 K 33/00,**
**A 61 K 33/06, A 61 K 33/14**
**//(A61K33/14, 33/06, 33/04)**

(54) **Médicament vétérinaire pour le traitement des jeunes veaux.**

(30) Priorité: **19.06.79 FR 7916184**

(43) Date de publication de la demande:
**07.01.81 Bulletin 81/1**

(45) Mention de la délivrance du brevet:
**25.05.83 Bulletin 83/21**

(84) Etats contractants désignés:
**AT CH DE GB LI LU SE**

(56) Documents cités:
**FR - A - 2 223 029**

**JEAN LECLERC "Formulaire Pharmaceutique",
1965, Vigot Freres, Paris FR Page 40
JEAN LECLERC "Formulaire Pharmaceutique",
1965, Vigot Freres, Paris FR Page 1048
JEAN LECLERC "Formulaire Pharmaceutique",
1965, Vigot Freres, Paris FR Page 1880**

**Le dossier contient des informations techniques
présentées postérieurement au dépôt de la
demande et ne figurant pas dans le présent
fascicule.**

(73) Titulaire: **Begouen, Jean-Paul
Lombost Archignat
F-03380 Huriel (FR)**

(72) Inventeur: **Begouen, Jean-Paul
Lombost Archignat
F-03380 Huriel (FR)**

(74) Mandataire: **Chanet, Jacques
B.P. 27 95bis avenue de Royat
F-63400 Chamalieres (FR)**

(56) Documents cités:
**REMINGTON'S PHARMACEUTICAL SCIENCES
(1975) pp. 864, 377, 378, 381
DICTIONNAIRE VIDAI (1963) "Magnéspasmyl"
p. 1146.**

Courier Press, Leamington Spa, England.

# 0 021 984

## Médicament vétérinaire pour le traitement des jeunes veaux

La présente invention a pour objet un médicament à usage vétérinaire, destiné au traitement des diarrhées dites "précoces" ou "blanches" des jeunes veaux.

On sait que les jeunes veaux peuvent être atteints dès les premiers jours de leur vie de troubles digestifs se caractérisant d'une manière générale par des selles abondantes blanches et très liquides; on sait aussi que le taux de mortalité consécutif à de telles affections est de l'ordre de quelques pourcents; on sait encore que du point de vue de l'étologie ces affections peuvent être d'origine infectieuse ou d'origine motrice, sans qu'il soit facile, pour autant, d'en distinguer, sans examen approfondi, la cause.

On a proposé de traiter la diarrhée d'origine motrice par des agents, tels que sélénium associé au tocophérol, destinés à rétablir la motricité normale de l'intestin, ou tels que cholinolytiques destinés à bloquer les sécrétions.

On a proposé de traiter la diarrhée d'origine infectieuse par des antiseptiques tels que des sulfamides, des dérivés de la quinoléïne et/ou par des antibiotiques tels que la collistine; à propos de ces derniers on a pu noter, l'antibio-résistance des souches infectieuses.

On sait enfin que des médications réhydratantes sont avantageusement combinées aux traitements précédents; de telles médications peuvent consister en l'administration par voie orale de grandes quantités (plusieurs litres) de solution d'électrolytes tels que chlorures alcalins ou alcalino-terreux.

Pour connaître de mainère plus précise l'art antérieur, on se réferera avantageusement à l'ouvrage de Jean Leclerc "Formulaire pharmaceutique, 1965, Vigot Frères, Paris" notamment aux chapitres ayant trait à l'acide nitrique (p. 40), aux sous-nitrates de bismuth (p. 1048) et à un sérum contre les diarrhées des veaux (p. 1880), on pourra se reférer encore au Rémington's Pharmaceutical Sciences (1975) dans ses articles relatifs au potassium, au lithium, au calcium, ainso qu'au "Dictionnaire Vidal" (1963) dans son article relatif à un médicament dénommé "magnéspasmyl".

La mise en oeuvre des traitements sus-visés réduit indéniablement la mortalité chez les jeunes veaux atteints de diarrhée; toutefois les médications élaborées sus-visées sont assez coûteuses et la décision de leur emploi nécessite de préférence le diagnostic d'un spécialiste.

La présente invention a pour objet un médicament peu coûteux et, semble-t-il, exempt d'effets secondaires préjudiciables aux animaux, elle résulte de l'observation des effets, sur un jeune veau atteint de diarrhée blanche, de l'absorption accidentelle d'une solution dont l'analyse a révélé les principes actifs mis en oeuvre dans une composition de l'invention.

Selon la présente invention une composition de traitement de la diarrhée des jeunes veaux consiste en une dose administrable oralement, ou "buvée", d'environ 1 litre d'une solution aqueuse, comportant environ 1 milliéquivalent/litre d'ions nitrates.

La composition est constituée par une solution aqueous de l'anion $NO_3^-$ à une concentration comprise entre 0,5 et 2 milliéquivalents par litre, au moins un des cations correspondants étant du groupe de cations comprenant les cations calcium, magnésium, sodium et potassium, tandis que le pH de ladite solution est ajusté à une valeur comprise entre 1 et 3; de préférence ladite solution comportera aussi des ions chlorures à raison d'environ un milliéquivalent/litre, et des ions sulfates dans une proportion de préférence moindre.

On notera d'une part que le taux de nitrates sus-visé est supérieur aux limites normalement tolérées par les animaux supérieurs et qu'il correspond à une eau chimiquement non potable pour l'home.

A titre indicatif, on a donné dans le tableau ci-dessous, un dosage ayant amené le retour normal des selles des jeunes veaux, moins de 24 heures après l'absorption d'un litre de solution, dans tous les cas de quinze cas de traitement, et le rétablissement des animaux dans les jours suivants.

| Nitrates | (en $NO_3^-$) | 1 mé/l | | Calcium | (en $Ca^{++}$) | mé/l 1,3 |
| Chlorures | (en $Cl^-$) | 1 mé/l | | Magnésium | (en $Mg^{++}$) | mé/l 0,6 |
| Sulfates | (en $SO_4^{--}$) | 0,30 mé/l | | Sodium | (en $Na^+$) | mé/l 0,4 |

La solution était préparée avec une eau silicieuse à raison d'environ 13 mg/l en $SiO_2$, ajustée à pH 1 à l'aide de l'acide nitrique ($NO_3H$).

Il doit être compris que les doses indiquées ci-dessus ne sont pas strictes mais qu'elles peuvent varier dans des plages allant de la moitié au double de ces valeurs, sans affecter sensiblement la santé de l'animal ni sans diminuer sensiblement l'efficacité du traitement.

Ainso la salinité totale exprimée en milliéquivalent de sel par litre pourra être comprise entre 1 et 5, elle contiendra notamment entre 0,5 et 2 milliéquivalent d'ions nitrate.

**0 021 984**

Exemple de préparation de la composition à partir de poudre

| | |
|---|---|
| Ca(NO$_3$)$_2$ . 4H$_2$O | 2 g |
| NaCl | 0,5 g |
| Na$_2$SO$_4$ . 10H$_2$O | 0,4 g |
| MgCl . 6H$_2$O | 0,3 g |
| | 3,2 g |

à dissoudre dans 10 litres d'eau et a administrer oralement par buvée de 1 litre toutes les vingt heures.

La composition de l'invention pourrait encore être présentée sous forme d'une solution des doses de poudres indiquées ci-dessus diluées dans 200 cc d'eau additionnée de 5 cc de NO$_3$H officinal, ce dernier composant étant destiné à abaisser aux environs de 1 le pH de la solution administrable, la solution concentrée pouvant être présentée en ampoule, flacon ou "berlingot", et étant alors destinée à être diluée dans 10 litres d'eau.

## Revendications

1. Composition vétérinaire destinée au traitement de la diarrhée des jeunes veaux, caractérisée:
   en ce qu'elle est constituée par une solution aqueuse de l'anion nitrate NO$_3^-$ à une concentration comprise entre 0,5 et 2 milliéquivalents par litre, au moins un des cations correspondants étant du groupe de cations comprenant les cations calcium, magnésium, sodium et potassium, et
   en ce que le pH de ladite solution est ajusté à une valeur comprise entre 1 et 3;
2. Composition selon la revendication 1, caractérisée:
   en ce que l'ajustement du pH de la solution est réalisé par adjonction d'acide nitrique;
3. Composition selon la revendication 2, caractérisée:
   en ce qu'elle comporte en outre l'anion chlorure présent à une concentration comprise entre 0,5 et 2 milliéquivalents par litre, et l'anion sulfate présent à une concentration comprise entre 0,15 et 0,6 milliéquivalent par litre;
4. Composition selon la revendication 1, caractérisé:
   en ce que le cation calcium est présent à la concentration comprise entre 0,65 et 2,6 milliéquivalents par litre, le cation magnésium à une concentration comprise entre 0,3 et 1,2 milliéquivalents par litre et le cation sodium à une concentration comprise entre 0,2 et 0,8 milliéquivalents par litre;
5. Composition selon la revendication 4, caractérisée:
   en ce que ladite composition est formée par la dissolution dans environ un litre d'eau de 0,2 g de nitrate de calcium tétra-hydraté (Ca(NO$_3$)$_2$ . 4H$_2$), de 0,05 g de chlorure de sodium (NaCl), de 0,04 g de sulfate de sodium déca-hydraté (Na$_2$SO$_4$ . 10H$_2$O) et 0,03 g de chlorure de magnésium hexa-hydraté (MgCl . 6H$_2$O).

## Patentansprüche

1. Veterinäre Verbindung zur Behandlung von Durchfall bei jungen Kälbern, dadurch gekennzeichnet, daß sie aus einer wässrigen Lösung von Nitrat-Anionen NO$_3^-$ bei einer Konzentration von zwischen 0,5 und 2 Milli-Äquivalenten pro Liter besteht, wobei wenigstens eine der entsprechenden Kationen von der Kationengruppe ist, die Kalzium-, Magnesium-, Natrium- und Kaliumkationen enthält, und daß der pH-Wert der genannten Lösung auf einen Wert eingestellt wird, der zwischen 1 und 3 liegt.
2. Verbindung nach Anspruch 1, dadurch gekennzeichnet, daß die Einstellung des pH-Wertes der Lösung durch Hinzufügen von Salpetersäure vorgenommen wird.
3. Verbindung nach Anspruch 2, dadurch gekennzeichnet, daß sie u.a. Chlorionen enthält, die in einer Konzentration zwischen 0,5 und 2 Milli-Äquivalenten pro Liter vorliegen, und daß das Sulfatanion in einer Konzentration von zwischen 0,15 und 0,6 Milli-Äquivalenten pro Liter vorliegt.
4. Verbindung nach Anspruch 1, dadurch gekennzeichnet, daß das Kalzium-Kation in einer zwischen 0,65 und 2,6 Milli-Äquivalenten pro Liter liegenden Konzentration vorlieft, das Magnesium-Kation eine Konzentration von zwischen 0,3 und 1,2 Milli-Äquivalenten pro Liter umfaßt, und das Natrium-Kation eine Konzentration von zwischen 0,2 und 0,8 Milli-Äquivalenten pro Liter.
5. Verbindung nach Anspruch 4, dadurch gekennzeichnet, daß die genannte Verbindung aus der Auflösung in ungefähr einem Liter Wasser von 0,2 g Kalzium-Tetra-Hydrat (Ca(NO$_3$)$_2$ . 4H$_2$), aus 0,05 g Natriumchlorid, aus 0,04 g Natriumsulfate Deca-Hydrat (Na$_2$SO$_4$ . 10H$_2$O) und aus 0,03 g Magnesiumchlorid Hexahydrate (MgCl . 6H$_2$O) gebildet ist.

## Claims

1. Veterinary composition intended for the treatment of diarrhoea in young calves, characterised

3

in that it comprises an aqueous solution of the $NO_3$ nitrate anion having a concentration of between 0.5 and 2 milliequivalents per litre, at least one of the corresponding cations being from the cation group comprising the calcium, magnesium, sodium and potassium cations, and in that the pH of the said solution is adjusted to a value between 1 and 3.

2. Composition according to claim 1, characterised in that the pH of the solution is adjusted by adding nitric acid.

3. Composition according to claim 2, characterised in that it also comprises the chloride anion which is present with a concentration of between 0.5 and 2 milliequivalents per litre, and the sulphate anion which is present with a concentration of between 0.15 and 0.5 milliequivalents per litre.

4. Composition according to claim 1, characterised in that the calcium cation present has a concentration of between 0.65 and 2.6 milliequivalents per litre, the magnesium cation a concentration of between 0.3 and 1.2 milliequivalents per litre and the sodium cation a concentration of between 0.2 and 0.8 milliequivalents per litre.

5. Composition according to claim 4, characterised in that the said composition is formed by dissolving, in approximately one litre of water, 0.2 g of calcium nitrate tetrahydrate ($Ca(NO_3)_2 . 4H_2O$), 0.05 of sodium chloride (NaCl), 0.04 g of sodium sulphate decahydrate ($Na_2SO_4 . 10H_2O$) and 0.03 g of magnesium chloride hexahydrate ($MgCl . 6H_2O$).